# EUROPEAN PATENT SPECIFICATION

(11) **EP 3 991 707 B1**
(45) Date of publication and mention of the grant of the patent: **01.05.2024**
(21) Application number: 20831576.2
(22) Date of filing: 19.06.2020
(51) Int. Cl.: A61F 13/49, A61F 13/494, A61F 13/505, A61F 13/74

(54) **ABSORBENT ARTICLE**
ABSORBIERENDER ARTIKEL
ARTICLE ABSORBANT

(30) Priority: 27.06.2019 JP 2019120419; 24.04.2020 JP 2020077367
(43) Date of publication of application: 04.05.2022
(73) Proprietor: KAO CORPORATION, Tokyo 103-8210 (JP)
(72) Inventor: SHIRAKAWA, Takashi, Haga-gun, Tochigi 321-3497 (JP); TOMITA, Mina, Haga-gun, Tochigi 321-3497 (JP)
(74) Representative: Vossius & Partner Patentanwälte Rechtsanwälte mbB
(86) International application number: PCT/JP2020/024051
(87) International publication number: WO 2020/262210

(56) References cited:
- WO-A1-2016/103866
- WO-A1-2017/110150
- JP-A- 2001 029 386
- JP-A- 2009 018 095
- JP-A- 2011 050 501
- JP-A- 2014 121 374
- JP-A- 2015 226 831
- JP-A- 2016 185 176

## Description

### Technical Field

The present invention relates to an absorbent article including leak-proof cuffs and leg cuffs.

### Background Art

Some absorbent articles such as disposable diapers include leak-proof cuffs also called three-dimensional gathers and three-dimensional guards. Typically, a pair of leak-proof cuffs are provided on the respective sides along the longitudinal direction of an assembly of an absorbent article (direction corresponding to the front-rear direction of a wearer). Each leak-proof cuff includes a leak-proof-cuff-forming sheet and elastic members fixed to the sheet in a stretched state and is to stand toward the skin of a wearer wearing the absorbent article to dam excrements such as feces, thus preventing lateral leakage. The absorbent article including the leak-proof cuffs typically further includes a pair of side flaps extending from the assembly outward in the lateral direction (direction orthogonal to the longitudinal direction) and also includes leg cuffs containing elastic members fixed in a stretched state in regions in the side flap corresponding to the legs of a wearer. The leg cuffs fitted onto the legs of a wearer can prevent undesirable excrement leakage along the legs.

Patent Literatures 1 to 3 disclose absorbent articles including leak-proof cuffs and leg cuffs, and each leak-proof cuff includes a base end portion at which a leak-proof-cuff-forming sheet is fixed to another member and a stand-up portion allowing the sheet to stand toward a wearer from the base end portion as a starting point. Each stand-up portion according to Patent Literatures 1 and 2 is folded along a folding portion extending in the longitudinal direction and includes an inner extension portion that extends inward in the lateral direction (direction orthogonal to the longitudinal direction) from the base end portion to the folding portion and an outer extension portion that extends outward in the lateral direction from the folding portion and is to be placed closer to the skin of a wearer than the inner extension portion. The stand-up portion according to Patent Literature 3 has a T-shape in a cross section along the lateral direction. Patent Literature 4 discloses a further absorbent article.

### Citation List

### Patent Literatures

Patent Literature 1: JP 11-285510 A
Patent Literature 2: JP 2011-50501 A
Patent Literature 3: JP 2008-307223 A
Patent Literature 4: JP2014121374

### Summary of Invention

The present invention relates to an absorbent article. The absorbent article has a longitudinal direction corresponding to the front-rear direction of a wearer and a lateral direction orthogonal to the longitudinal direction, and includes: an absorbent assembly including an absorbent member; a pair of leak-proof cuffs extending in the longitudinal direction and provided on a skin-facing surface of the absorbent assembly while a gap is interposed therebetween; side sheets extending outward in the lateral direction from side edges along the longitudinal direction of the absorbent member; and a pair of leg cuffs provided outside the pair of leak-proof cuffs in the lateral direction and in regions corresponding to the legs of a wearer.

In an embodiment of the absorbent article of the present invention, each of the pair of leak-proof cuffs includes the side sheet, and has, in the lateral direction, a base end portion at which the side sheet is fixed to another member and a stand-up portion that is a portion of the side sheet and stands toward a wearer from the base end portion as a starting point.

In an embodiment of the absorbent article of the present invention, the stand-up portion is folded along a folding portion extending in the longitudinal direction and includes a first stand-up portion extending from the base end portion to the folding portion and a second stand-up portion extending from the folding portion to a free end of the stand-up portion and to be placed closer to the skin of a wearer than the first stand-up portion; and in the second stand-up portion, a plurality of leak-proof-cuff-forming elastic members are arranged in the lateral direction in a stretchable state in the longitudinal direction.

In an embodiment of the absorbent article of the present invention, the leg cuff includes the side sheet and a plurality of leg-cuff-forming elastic members provided on the side sheet; and the plurality of leg-cuff-forming elastic members are arranged in the lateral direction in a stretchable state in the longitudinal direction and located outside the base end portion in the lateral direction.

In an embodiment of the absorbent article of the present invention, the contractive force per one leak-proof-cuff-forming elastic member in the second stand-up portion at a cuff longitudinal length of 70 is smaller than the contractive force per one leg-cuff-forming elastic member in the leg cuff at a cuff longitudinal length of 70, and the contractive force of the whole second stand-up portion at a cuff longitudinal length of 70 is larger than the contractive force of the whole leg cuff at a cuff longitudinal length of 70, where the cuff longitudinal length is a length of each of the leak-proof cuff and the leg cuff in the longitudinal direction, and the cuff longitudinal length of the leg cuff and the cuff longitudinal length of the leg cuff in a fully stretched state are each defined as 100.

Other features, effects, and embodiments of the present invention will be described below.

### Brief Description of Drawings

[Fig. 1] Fig. 1 is a partially broken, developed plan view schematically showing a skin-facing surface side (topsheet side) of an open type disposable diaper in a spread out and stretched state as an embodiment of the absorbent article of the present invention.
[Fig. 2] Fig. 2 is a schematic cross-sectional view showing a cross section taken along line I-I in Fig. 1.
[Fig. 3] Fig. 3 is an example of a graph showing the relation between the cuff longitudinal length and the contractive force, for a whole second stand-up portion in a leak-proof cuff and a whole leg cuff.
[Fig. 4] Fig. 4 is an example of a graph showing the relation between the cuff longitudinal length and the contractive force per one elastic member, for a second stand-up portion in a leak-proof cuff and of a leg cuff.
[Fig. 5] Fig. 5 is an example of a graph showing the relation between the cuff longitudinal length and the contractive force, for the laterally innermost part and the laterally outermost part of a second stand-up portion in a leak-proof cuff.

### Description of Embodiments

Conventional absorbent articles including leak-proof cuffs and leg cuffs have room for improvement in the capability of the leak-proof cuffs to prevent leakage and also in well-fitting of the leg cuffs. The present invention relates to an absorbent article that includes leak-proof cuffs having strong capability to prevent lateral leakage and leg cuffs achieving well-fitting and thus can effectively prevent lateral leakage.

The present invention will now be described on the basis of preferred embodiments thereof with reference to drawings. In the following description of drawings, identical or similar components are indicated by an identical or similar sign. Drawings are basically schematic, and dimensional ratios and so on may differ from the actual ones.

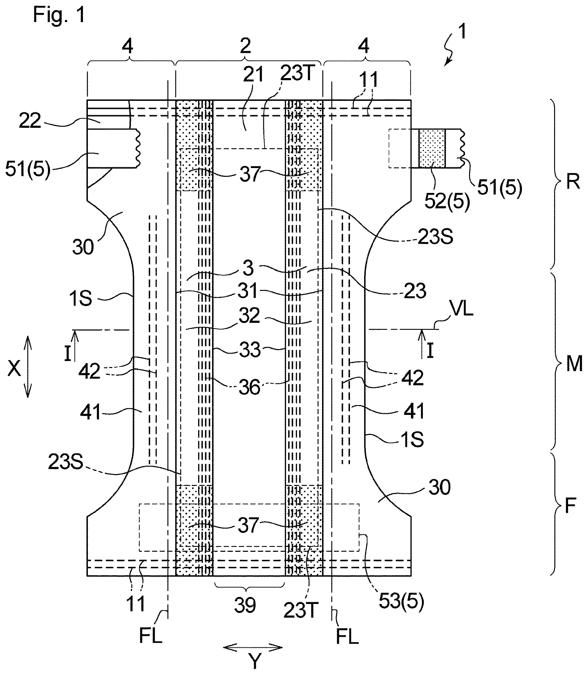

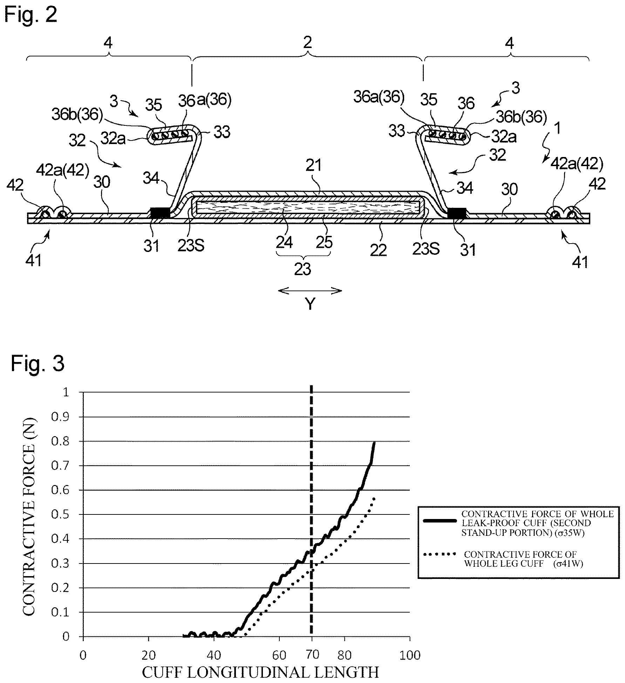

Fig. 1 and Fig. 2 show a disposable diaper 1 as an embodiment of the absorbent article of the present invention. The diaper 1 has a longitudinal direction X corresponding to the front-rear direction of a wearer (i.e., a direction extending from the front side through the crotch to the rear side), and a lateral direction Y orthogonal thereto, and includes: an absorbent assembly 2 including an absorbent member 23; a pair of leak-proof cuffs 3, 3 extending in the longitudinal direction X and provided on a skin-facing surface of the absorbent assembly 2 while a gap 39 is interposed therebetween; side sheets 30 extending outward in the lateral direction Y from the respective side edges 23S, 23 S along the longitudinal direction X of the absorbent member 23; and a pair of leg cuffs 41, 41 provided outside the pair of leak-proof cuffs 3,3 in the lateral direction Y and in regions corresponding to the legs of a wearer. The diaper 1 shown in the figures includes a pair of side flaps 4, 4 provided at the respective sides of the absorbent assembly 2 in the lateral direction Y and including the side sheets 30, and the pair of leg cuffs 41, 41 are provided in the respective side flaps 4.

Herein, a "skin-facing surface" is one face of an absorbent article or a constituent member thereof (for example, an absorbent assembly) and is a face facing the skin of a wearer at the time of wearing the absorbent article, and in other words, the face relatively close to the skin of a wearer. A "non-skin-facing surface" is the other face of an absorbent article or a constituent member thereof and is a face facing the opposite direction from the skin of a wearer at the time of wearing the absorbent article, and in other words, is the face relatively distant from the skin of a wearer. Herein, "at the time of wearing" means a state in which a typical, appropriate wearing position or a normal wearing position of the absorbent article is maintained.

The diaper 1 (absorbent assembly 2) of the present embodiment, as shown in Fig. 1, includes: a crotch portion M to be placed on the crotch of a wearer wearing the diaper; a front portion F to be placed on the anterior side to the crotch portion M in the longitudinal direction X, i.e., on the front side of the wearer; and a rear portion R to be placed on the posterior side to the crotch portion M in the longitudinal direction X, i.e., on the rear side of the wearer. The crotch portion M includes an excretory-facing portion (not shown) to be placed to face the excretory such as the penis of a wearer wearing the diaper.

The absorbent assembly 2 includes the absorbent member 23, a topsheet 21 placed on a skin-facing surface of the absorbent member 23, and a backsheet 22 placed on a non-skin-facing surface of the absorbent member 23, and these members are integrated by a known joining means such as an adhesive to form the absorbent assembly. The absorbent member 23 is interposed between the topsheet 21 and the backsheet 22 and includes a liquid-retentive absorbent core 24 containing a water absorbing material and a core-wrap sheet 25 overlying the outer surface (the skin-facing surface and the non-skin-facing surface) of the absorbent core 24. The absorbent assembly 2 of the present embodiment has a rectangular shape in a planar view and extends in the longitudinal direction X from the front portion F to the rear portion R, and the length direction thereof is coincident with the longitudinal direction X.

As each of the topsheet 21, the backsheet 22, and the absorbent member 23, various materials conventionally used in such absorbent articles can be used without any limitation. As the topsheet 21, for example, a single layer or multilayer nonwoven fabric or porous film having liquid permeability can be used. As the backsheet 22, a waterproof sheet, that is, a sheet having liquid impermeability (no liquid passes therethrough) or having poor liquid permeability (liquid is not completely prevented from passing therethrough but is difficult to pass) can be used, and, for example, a moisture permeable resin film or laminate of a resin film and a nonwoven fabric can be used. As the absorbent core 24 included in the absorbent member 23, for example, an airlaid fiber product prepared by air-laying a water absorbing material such as wood pulp and a water absorbing polymer, or a sheet-like absorbing structure containing the water absorbing material can be used. As the core-wrap sheet 25 included in the absorbent member 23, a liquid permeable sheet can be used, and, for example, paper or a nonwoven fabric can be used.

In the present embodiment, as shown in Fig. 2, the topsheet 21 covers the whole region on the skin-facing surface of the absorbent member 23, whereas the backsheet 22 covers the whole region on the non-skin-facing surface of the absorbent member 23, and both the sheets 21, 22 further extend outward in the lateral direction Y from the respective side edges 23 S, 23 S of the absorbent member 23 to form the side flaps 4 together with the side sheets 30. As described above, the side flaps 4 in the present embodiment are located outside the absorbent member 23 in the lateral direction Y and include the topsheet 21, the backsheet 22, and the side sheets 30. These members included in the side flaps 4 are joined together by a known joining means such as an adhesive, heat sealing, and ultrasonic sealing.

In each of the end portion of the front portion F and that of the rear portion R in the longitudinal direction X, that is, in each waist end portion, waist gather-forming elastic members 11 are provided in a stretchable state in the lateral direction Y and extend in the lateral direction Y from one side flap 4 to the other side flap 4. The waist gather-forming elastic members 11 are fixed in a stretched state in the lateral direction Y between the topsheet 21 and the backsheet 22 that extend outward in the longitudinal direction X from the respective longitudinal ends 23T, 23T of the absorbent member 23. With such a configuration, the waist end portions can be contracted by a contraction force of the waist gather-forming elastic members 11 to fit around the waist of a wearer wearing the diaper 1.

The diaper 1 is what is called an open type disposable diaper and includes an attachment structure 5 to be used at the time of wearing. As shown in Fig. 1, the attachment structure 5 includes a pair of fastening tapes 51, 51 provided on the respective side edge portions along the longitudinal direction X in the rear portion R of the diaper 1 and a target region 53 provided on the non-skin-facing surface in the front portion F of the diaper 1 (the non-skin-facing surface of the backsheet 22). Each fastening tape 51 has an attachment portion 52, and the attachment portions 52 are attached to the target region 53 when the diaper 1 is worn. Onto the target region 53, the attachment portions 52 can be attached detachably. Typically, the attachment portion 52 includes a male member of a hook and loop fastener, whereas the target region 53 includes a female member of the hook and loop fastener.

As shown in Fig. 1 and Fig. 2, each of the pair of leak-proof cuffs 3, 3 includes the side sheet 30 and has, in the lateral direction Y, a base end portion 31 at which the side sheet 30 is fixed to another member, and a stand-up portion 32 that is a portion of the side sheet 30 and stands toward a wearer from the base end portion 31 as a starting point.

As shown in Fig. 1 and Fig. 2, a pair of the side sheets 30 are provided in the respective side portions along the longitudinal direction X, on the skin-facing surface of the absorbent assembly 2, and there is a gap 39 between the pair of side sheets 30, 30. The gap 39 is located at the center portion of the absorbent assembly 2 (diaper 1) in the lateral direction Y Each side sheet 30 is provided over the corresponding side edge 23S of the absorbent member 23 in the lateral direction Y and includes, in the lateral direction Y, a portion overlapping with the absorbent member 23 and a portion located outside the absorbent member 23 in the lateral direction Y in a plan view.

As the side sheet 30, a hydrophobic sheet material is used in view of reliably fulfilling such a role as to prevent leakage of body fluids such as urine to the outside. As the hydrophobic sheet material, a hydrophobic nonwoven fabric can be used, for example, and specifically, an air-through nonwoven fabric, a spunbonded nonwoven fabric, an SMS nonwoven fabric, an SMMS nonwoven fabric, or an SSMS nonwoven fabric can be used. A hydrophobic resin film, a laminate of such a resin film and the above-described nonwoven fabric, or the like can also be used. Herein, "S" means spunbonded nonwoven fabric, and "M" means meltblown nonwoven fabric. The side sheet 30 as a leak-proof-cuff-forming sheet preferably has a water-pressure resistance of 80 mmHzO or more and particularly 100 mmHzO or more as determined in accordance with ISO 811.

The base end portion 31 is a starting point of the standing when the stand-up portion 32 stands toward the skin of a wearer wearing the diaper 1. More specifically, the base end portion 31 is in a fixed part of the side sheet 30 to "another member" and is a portion adjacent to the stand-up portion 32 in the lateral direction Y, and in other words, the base end portion 31 is the innermost portion of the fixed part in the lateral direction Y The "another member" is a constituent member of the diaper 1 except the side sheet 30. The "another member" is typically a member that is provided on the non-skin-facing surface side of the side sheet 30 and can be in contact with the side sheet 30, and is the topsheet 21 in the present embodiment as shown in Fig. 2.

The base end portion 31 is a fixed part between the side sheet 30 and another member, as described above, and is typically formed by a known joining means such as an adhesive including hot melt adhesive and fusion bonding. The base end portion 31 in the present embodiment has a straight line shape in a plan view and extends in the longitudinal direction X over substantially the entire length in the longitudinal direction X of the side sheet 30, as shown in Fig. 1, and also the base end portion 31 is located outside the side edge 23S of the absorbent member 23 in the lateral direction Y and near the side edge 23S, as shown in Fig. 2. The planar shape of the base end portion 31 is not specifically limited and can be a straight line, a wavy line, a curved line, or a zigzag line, for example. The base end portion 31 does not necessarily continue in one direction (longitudinal direction X) and may be a broken line composed of fixed parts and non-fixed parts between the side sheet 30 and another member, the fixed parts and non-fixed parts alternately arranged in the longitudinal direction X.

The stand-up portion 32 is a non-fixed part in the side sheet 30 to another member. As shown in Fig. 1 and Fig. 2, the stand-up portion 32 is folded along a folding portion 33 extending in the longitudinal direction X, and includes a first stand-up portion 34 extending from the base end portion 31 to the folding portion 33 and a second stand-up portion 35 extending from the folding portion 33 to a free end 32a of the stand-up portion 32 and to be placed closer to the skin of a wearer than the first stand-up portion 34. The surface of the second stand-up portion 35 can be in contact with the skin of a wearer wearing the diaper 1.

In the present embodiment, as shown in Fig. 2, the stand-up portion 32 is folded outside in the lateral direction Y Accordingly, the first stand-up portion 34 included in the stand-up portion 32 extends inward in the lateral direction Y from the base end portion 31 to the folding portion 33, and the second stand-up portion 35 included in the stand-up portion 32 extends outward in the lateral direction Y from the folding portion 33. The folding portion 33 is the nearest portion to the center of the diaper 1 in the lateral direction Y in the stand-up portion 32 and has a straight line shape extending in the longitudinal direction X in the diaper 1 in such a spread out and stretched state as shown in Fig. 1. The gap 39 between the pair of leak-proof cuffs 3, 3 is a region between the folding portion 33 of one leak-proof cuff 3 and the folding portion 33 of the other leak-proof cuff 3. The "spread out and stretched state of a diaper 1 (absorbent article)" is a state in which the diaper 1 is spread out as shown in Fig. 1 and flattened out to the design size (equal to the size of a flattened diaper in the state where the effect of the elastic members is completely eliminated) by stretching the elastic members. In a diaper 1 in a spread out and stretched state, typically the stand-up portions 32 do not stand but fall; however, in Fig. 2, which shows the spread out and stretched state, the stand-up portions 32 stand for the sake of simple description.

In the second stand-up portion 35, a plurality of (specifically four) leak-proof-cuff-forming elastic members 36 are arranged in the lateral direction Y in a stretchable state in the longitudinal direction X, as shown in Fig. 1 and Fig. 2. In the second stand-up portion 35, a portion in which the leak-proof-cuff-forming elastic members 36 are fixed in a stretchable state in the longitudinal direction X has elasticity in the longitudinal direction X. More specifically, the second stand-up portion 35, as shown in Fig. 2, includes a portion in which the side sheet 30 is folded in half in the lateral direction Y into a double layer structure, and between the two sheets 30, 30 constituting the double layer structure, a plurality of (four) filamentous leak-proof-cuff-forming elastic members 36 are arranged in the lateral direction Y and fixed in a stretched state in the longitudinal direction X. Accordingly, each elastic member 36 is provided in a stretchable state in the longitudinal direction X. The free end 32a of the stand-up portion 32 is on the folding line of the side sheet 30 folded in half. The leak-proof cuff forming elastic member 36 is preferably filamentous, or alternatively may be a flat band. The number of the leak-proof-cuff-forming elastic members 36 is not specifically limited as long as it is at least two.

The leak-proof cuff 3 is typically prepared by the following procedure: leak-proof-cuff-forming elastic members 36 are fixed in a stretched state to a side sheet 30; and then the stretched state is released. Due to such a preparation method, many creases are formed on the surface of the second stand-up portion 35, which can be in contact with the skin of a wearer wearing the diaper 1. The creases on the surface of the second stand-up portion 35 are intermittently formed substantially regularly in the longitudinal direction X.

The stand-up portions 32 are present preferably in at least the crotch portion M. This is because the diaper 1 has the problem of lateral leakage particular in the crotch portion M, in which excrements are mainly discharged. As shown in Fig. 1, the stand-up portions 32 in the present embodiment continue over the entire length of the crotch portion M in the longitudinal direction X and further extend to both the front portion F and the rear portion R.

As shown in Fig. 1, stand-up inhibitory portions 37 are formed in the respective longitudinal outer regions relative to the stand-up portions 32 of the pair of leak-proof cuffs 3, 3. The stand-up portion 32 is interposed between a stand-up inhibitory portion 37 at one end in the longitudinal direction X (in the front portion F) and a stand-up inhibitory portion 37 at the other end (in the rear portion R). In the stand-up inhibitory portion 37, a portion corresponding to the stand-up portion 32 in the lateral direction Y (i.e., the non-fixed part to another member) in the side sheet 30 is fixed to another member (in the present embodiment, the topsheet 21) in the position overlapping with the absorbent member 23 in a plan view. This prevents standing-up of the stand-up inhibitory portions 37 even when the stand-up portions 32 stand.

At the time of wearing the diaper 1, the stand-up portion 32 (the first stand-up portion 34 and the second stand-up portion 35) of each of the pair of leak-proof cuffs 3, 3 stands, due to a contraction force of the leak-proof-cuff-forming elastic members 36 provided in the second stand-up portion 35, from the base end portion 31 as a stand-up base end, as shown in Fig. 2. Accordingly, a pair of leak-proof cuffs are formed by the stand-up portions 32 on the respective sides along the longitudinal direction X, in at least the crotch portion M on the skin-facing surface of the absorbent assembly 2. The pair of leak-proof cuffs dam excrements such as urine and feces excreted on the skin-facing surface of the diaper 1 and prevent the excrements from leaking outside the diaper 1 in the lateral direction Y, i.e., prevent what is called lateral leakage.

At the time of wearing the diaper 1, the diaper 1 is curved by a contraction force of the leak-proof cuff forming elastic members 36 in such a way that the longitudinal center portion of the diaper 1 (a portion including the crotch portion M), that is, a portion with the elastic members 36, forms a convex on the non-skin-facing surface (on the backsheet 22), and in other words, forms a concave on the skin-facing surface (on the topsheet 21). Accordingly, the diaper 1 is deformed into a boat shape as a whole. Such a boat-shaped diaper 1 easily fits with the crotch shape of a wearer. Hence, the second stand-up portions 35 of the stand-up portions 32 in a standing state in the diaper 1 come into close contact with the skin of a wearer with well-fitting, and accordingly, the effect of preventing lateral leakage can be successfully exhibited by the leak-proof cuffs 3.

Each of the pair of leg cuffs 41, 41 includes the side sheet 30 and a plurality of leg-cuff-forming elastic members 42 provided on the side sheet 30. As shown in Fig. 2, the leg cuff 41 in the present embodiment further includes the backsheet 22 and two filamentous elastic members as the leg-cuff-forming elastic members 42. The plurality of (two) filamentous leg-cuff-forming elastic members 42 are arranged in the lateral direction Y in a stretchable state in the longitudinal direction X and located outside the base end portion 31 of the leak-proof cuff 3 in the lateral direction Y Each of the plurality of leg-cuff-forming elastic members 42 is fixed to a sheet-like member included in the side flap 4, such as the side sheet 30, in a stretched state in the longitudinal direction X at least over the entire length of the crotch portion M in the longitudinal direction X and thus is in a stretchable state in the longitudinal direction X. With such a configuration, the leg cuffs 41 can be contracted by a contraction force of the leg-cuff-forming elastic members 42 to fit around the legs of a wearer wearing the diaper 1. The leg-cuff-forming elastic members 42 are preferably filamentous, or alternatively may be a flat band. The number of the leg-cuff-forming elastic members 42 is not specifically limited as long as it is at least two.

The diaper 1 is characterized by satisfying both the following conditions (A) and (B), where the length of the leak-proof cuff 3 (second stand-up portion 35) and that of the leg cuff 41 in the longitudinal direction X are each referred to as a "cuff longitudinal length", and the cuff longitudinal length of the cuff 3 and that of the cuff 41 in a fully stretched state are each defined as 100. As clear from the above, the "cuff longitudinal length" is a relative longitudinal length of a cuff to the longitudinal direction of the cuff in a fully stretched state as a reference value. The "cuff longitudinal length" is typically minimum when the cuff is in a natural state (no external force is applied), but the minimum length is not zero.
(A) The contractive force per one leak-proof-cuff-forming elastic member 36 in the second stand-up portion 35 at a cuff longitudinal length of 70, σ₇₀35, is smaller than the contractive force per one leg-cuff-forming elastic member 42 in the leg cuff 41 at a cuff longitudinal length of 70, σ₇₀41.
(B) The contractive force of the whole second stand-up portion 35 at a cuff longitudinal length of 70, σ₇₀35W, is larger than the contractive force of the whole leg cuff 41 at a cuff longitudinal length of 70, σ₇₀41W.

Each contractive force was determined by the following method on a measurement sample prepared by the following method.

### <Preparation of measurement sample>

First, a diaper 1 (absorbent article) is cut over the whole length in the longitudinal direction X into a first section including two types of cuffs (a leak-proof cuff 3 (a second stand-up portion 35), a leg cuff 41) to be compared. To prepare the first section, a diaper 1 can be bisected in the lateral direction Y, for example. The first section includes: a second stand-up portion 35 (a portion from a folding portion 33 to a free end 32a) of a leak-proof cuff 3; extended regions when the second stand-up portion 35 is virtually extended in the longitudinal direction X toward both ends (in the diaper 1 shown in Fig. 1, the stand-up inhibitory portions 37); a leg cuff 41; and extended regions when the leg cuff 41 is virtually extended in the longitudinal direction X toward both ends.

Next, in the first section, a portion in which elastic members (leak-proof-cuff-forming elastic members 36, leg-cuff-forming elastic members 42) are provided in a stretched state in the longitudinal direction X is cut to give a second section having a length of 100 mm in the longitudinal direction X in a spread out and stretched state and having a quadrangular shape in a plan view. The second section corresponds to a portion having elasticity in the longitudinal direction X in the diaper 1 and is typically a portion corresponds to the crotch portion M.

The second section is then cut in the lateral direction Y along the respective elastic members provided in the second section to give the same number of measurement samples as the number of the elastic members contained in the second section. Each of the plurality of measurement samples prepared as above includes a single elastic member (a leak-proof-cuff-forming elastic member 36 or a leg-cuff-forming elastic member 42) and a sheet to which the elastic member is fixed and has a longitudinal length (length in the longitudinal direction X) of 100 mm. When the second section is cut out from the diaper 1 shown in Fig. 1, the sheet is a side sheet 30 in a measurement sample containing a leak-proof-cuff-forming elastic member 36 as the elastic member, whereas the sheet is a side sheet 30 and a backsheet 22 in a measurement sample containing a leg-cuff-forming elastic member 42 as the elastic member.

### <Measurement method of contractive force>

The respective longitudinal ends of a measurement sample are clamped to chucks of a Tensilon universal tester (RTC-1210A) manufactured by ORIENTEC. Then, the measurement sample is stretched in the length direction (longitudinal direction X) to the fully stretched state by expanding the chuck distance at a speed of 300 mm/min, and the measurement sample is contracted to a predetermined length by reducing the chuck distance at a speed of 300 mm/min. The force (contractive force) (unit: N) during the contracting is determined. As described above, each force σ₇₀35, σ₇₀41 to be determined is a contractive force at a relative length of 70 to the cuff longitudinal length in a fully stretched state, which is defined as 100. Hence, in the above measurement, a measurement sample is contracted from a fully stretched state corresponding to a cuff longitudinal length of 100 to a length corresponding to a cuff longitudinal length of 70. The respective contractive forces of the plurality of measurement samples prepared from a leak-proof cuff 3 (second stand-up portion 35) are determined. The average of these found values is calculated and used as "contractive force σ₇₀35" (contractive force per one leak-proof-cuff-forming elastic member 36 in a second stand-up portion 35 at a cuff longitudinal length of 70), and the sum of these found values is calculated and used as "contractive force σ₇₀35W" (contractive force of a whole second stand-up portion 35 at a cuff longitudinal length of 70). Separately, the respective forces of the plurality of measurement samples prepared from a leg cuff 41 are determined. The average of these found values is calculated and used as "contractive force σ₇₀41" (contractive force per one leg-cuff-forming elastic member 42 in a leg cuff 41 at a cuff longitudinal length of 70), and the sum of these found values is calculated and used as "force σ₇₀41W" (contractive force of a whole leg cuff 41 at a cuff longitudinal length of 70).

For example, in the embodiment shown in Fig. 2, four leak-proof-cuff-forming elastic members 36 are provided in the second stand-up portion 35, and thus four measurement samples corresponding to the four elastic members 36 are prepared from the second stand-up portion 35 by the above method. The respective contractive forces of the four measurement samples having a length corresponding to a cuff longitudinal length of 70 are determined. The average of the four found values is calculated to give a contractive force σ₇₀35, and the sum of the four found values is calculated to give a contractive force σ₇₀35W. In the embodiment shown in Fig. 2, two leg-cuff-forming elastic members 42 are provided in the leg cuff 41, and thus two measurement samples corresponding to the two elastic members 42 are prepared from the leg cuff 41 by the above method. The respective contractive forces of the two measurement samples having a length corresponding to a cuff longitudinal length of 70 are determined. The average of the two found values is calculated to give a contractive force σ₇₀41, and the sum of the two found values is calculated to give a contractive force σ₇₀41W.

As described above, each force (σ₇₀35, σ₇₀35W, σ₇₀41, σ₇₀41W) is a contractive force of the corresponding cuff at a cuff longitudinal length of 70, where the length of the cuff (the second stand-up portion 35 of the leak-proof cuff 3, the leg cuff 41) in a fully stretched state in the longitudinal direction X (cuff longitudinal length) is defined as 100. The "cuff longitudinal length of 70" corresponds to a normal longitudinal length of the leak-proof cuff or the leg cuff when this kind of absorbent article (disposable diaper) is worn. The reason why the force of interest is not a force when the cuff is being stretched (stretching force) but is a force when the cuff is being contracted from a fully stretched state (contractive force) is that the operation of a carer who puts a diaper on a subject person is considered. In other words, when a diaper is put on, each portion of the diaper is typically stretched, and the diaper is then allowed to fit on the body shape and thus is deformed from a stretched state to a contracted state. To follow the deformation of the diaper when the diaper is put on, a measurement sample is deformed from a stretched state to a contracted state, and the contractive force during the contracting is determined, in the above measurement method.

The technical importance of the magnitude relation "contractive force σ₇₀35 < contractive force σ₇₀41" provided in the condition (A) is mainly that the contractive force (σ₇₀35) per one leak-proof-cuff-forming elastic member 36 included in the second stand-up portion 35 to be in contact with the skin of a wearer wearing the diaper 1 is reduced to prevent marks due to compression by the elastic members 36 from being left on the skin of the wearer of the diaper 1. However, if the whole second stand-up portion 35 had an excessively low contractive force, the second stand-up portion 35 would be unlikely to fit with the skin of a wearer, resulting in reduction in the capability of the leak-proof cuff 3 to prevent lateral leakage. Hence, the diaper 1 further satisfies the condition (B) in addition to the condition (A). In other words, by satisfying the relation, contractive force σ₇₀35 < contractive force σ₇₀41, marks due to compression by the leak-proof-cuff-forming elastic members 36 are unlikely to be formed on the skin of a wearer, and by satisfying the relation, contractive force σ₇₀35W > contractive force σ₇₀41W, the whole second stand-up portion 35 does not have an excessively low contractive force. The second stand-up portions 35 of the leak-proof cuffs 3 of the diaper 1 are in close contact with the skin of a wearer with well-fitting at the time of wearing, with an appropriate compression force that does not left marks of the leak-proof-cuff-forming elastic members 36, and thus lateral leakage can be effectively prevented.

In the leak-proof cuff 3 shown in Fig. 2, the first stand-up portion 34 extends inward in the lateral direction Y from the base end portion 31 to the folding portion 33, and the second stand-up portion 35 extends outward in the lateral direction Y from the folding portion 33. When the conditions (A) and (B) are satisfied in such a configuration, the diaper 1 has the following feature when worn. Specifically, with such an embodiment as above, at a cuff longitudinal length of 70, the contractive force of the leg cuff 41 is unlikely to cause contraction of the leak-proof cuff 3, and thus disadvantages is unlikely to be caused, including impairment of standing-up behavior of the leak-proof cuff 3 and irregular creases in the second stand-up portion 35. Consequently, the close contact between the skin of a wearer and the surface of the second stand-up portions 35 is easily maintained, and lateral leakage of excreted fluids can be more effectively prevented.

At a cuff longitudinal length of 70, the laterally outermost leak-proof-cuff-forming elastic member 36b in the second stand-up portion 35 of the leak-proof cuff 3 is preferably located laterally inside the laterally innermost leg-cuff-forming elastic member (indicated by sign 42a in the drawing) of the plurality of leg-cuff-forming elastic members 42 as shown in Fig. 2, when the diaper 1 is worn. The positional relation between the elastic members 36b, 42a described here is applied to a case in which the elastic members 36b, 42a are located on the same side when the diaper 1 is bisected in the lateral direction Y (for example, on the right side or the left side of the lateral center of the diaper 1 as the boundary, in Fig. 2). Even when the contractive force per one leg-cuff-forming elastic member 42 is larger than that per one leak-proof-cuff-forming elastic member 36 at a cuff longitudinal length of 70, that is, even when the diaper 1 satisfies the condition (A), such a configuration as described above further suppresses the influence of the condition (A) on the standing-up behavior of the first stand-up portion 34 of the leak-proof cuff 3, and thus, in addition to the advantageous effect by the conditions (A) and (B), the close contact between the skin of a wearer and the leak-proof cuffs 3 is easily maintained to more effectively prevent lateral leakage of excreted fluids advantageously.

Fig. 3 is an example graph showing the relation between the cuff longitudinal length and the contractive force (σ35W, σ41W), for the whole second stand-up portion 35 of a leak-proof cuff 3 and a whole leg cuff 41. In the present invention, as provided in the condition (B), the contractive forces of the whole cuffs 3, 41 satisfy the magnitude relation "the contractive force of a second stand-up portion 35 > the contractive force of a leg cuff 41" at least at a cuff longitudinal length of 70. As for the relation, the magnitude relation is constantly satisfied regardless of the cuff longitudinal length, in the example shown in Fig. 3. The present invention also encompasses the embodiment in which the magnitude relation of contractive force is as shown by the graph in Fig. 3, and in other words, an embodiment in which the relation "contractive force σ₇₀35W > contractive force σ₇₀41W" is satisfied not only when the cuff longitudinal length is 70 but also when the cuff longitudinal length is not 70.

The ratio of the contractive force σ₇₀35 to the contractive force σ₇₀41 (σ₇₀35/σ₇₀41) is preferably 0.2 or more and more preferably 0.3 or more and is preferably 0.99 or less and more preferably 0.9 or less.

The contractive force σ₇₀35 (contractive force per one leak-proof-cuff-forming elastic member 36 in a second stand-up portion 35 at a cuff longitudinal length of 70) is preferably 0.03 N or more and more preferably 0.06 N or more and is preferably 0.15 N or less and more preferably 0.12 N or less.

The contractive force σ₇₀41 (contractive force per one leg-cuff-forming elastic member 42 in a leg cuff 41 at a cuff longitudinal length of 70) is preferably 0.04 N or more and more preferably 0.08 N or more and is preferably 0.24 N or less and more preferably 0.20 N or less.

The ratio of the contractive force σ₇₀35W to the contractive force σ₇₀41W (σ₇₀35W/σ₇₀41W) is preferably more than 1 and more preferably 1.25 or more and is preferably 6 or less and more preferably 5 or less.

The contractive force σ₇₀35W (contractive force of a whole second stand-up portion 35 at a cuff longitudinal length of 70) is preferably 0.1 N or more and more preferably 0.2 N or more and is preferably 0.6 N or less and more preferably 0.5 N or less.

The contractive force σ₇₀41W (contractive force of a whole leg cuff 41 at a cuff longitudinal length of 70) is preferably 0.05 N or more and more preferably 0.1 N or more and is preferably 0.5 N or less and more preferably 0.4 N or less.

Both the conditions (A) and (B) can be satisfied by making the leak-proof-cuff-forming elastic members 36 and the leg-cuff-forming elastic members 42 different in, for example, at least one of the type (the composition of material), the thickness, the stretch ratio, and the joining length (length of a portion in the elastic member joined to another member with an adhesive or the like). For satisfying the relation, contractive force σ₇₀35 < contractive force σ₇₀41, a leak-proof-cuff-forming elastic member 36 may have a smaller diameter than a leg-cuff-forming elastic member 42, for example. In such a condition, a larger number of the leak-proof-cuff-forming elastic members 36 may be provided in the second stand-up portion 35 than the number of the leg-cuff-forming elastic members 42 provided in the leg cuff 41, for also satisfying the relation, contractive force σ₇₀35W > contractive force σ₇₀41W. On both one side and the other side about an imaginary lateral center line (not shown) bisecting the diaper 1 in the embodiment shown in Fig. 2, the number of the leak-proof-cuff-forming elastic members provided in the second stand-up portion is 4, whereas the number of the leg-cuff-forming elastic members is 2, and the former is more than the latter.

In addition to satisfying both the conditions (A) and (B), the magnitude relation between the contractive force per one leak-proof-cuff-forming elastic member 36 in the second stand-up portion 35, σ35, and the contractive force per one leg-cuff-forming elastic member 42 in the leg cuff 41, σ41, within a cuff longitudinal length range of less than a particular value preferably differs from that within a cuff longitudinal length range of more than the particular value and not more than 100. In other words, the following condition is preferable: when the cuff longitudinal length is in a range of not less than 0 and less than a particular value, one of the magnitude relations of "contractive force σ35 < contractive force σ41" and "contractive force σ35 > contractive force σ41" is satisfied, and when the cuff longitudinal length is in a range of more than the particular value and not more than 100, the other magnitude relation is satisfied. Satisfying such a condition leads to the following advantage: when the cuff longitudinal length is relatively small, one having a larger contractive force among the leak-proof cuff 3 (second stand-up portion 35) and the leg cuff 41 contracts before the other contracts, and comes into close contact with the skin of a wearer with well-fitting, which couples with the advantageous effect by satisfying both the conditions (A) and (B) to prevent the lateral leakage more effectively.

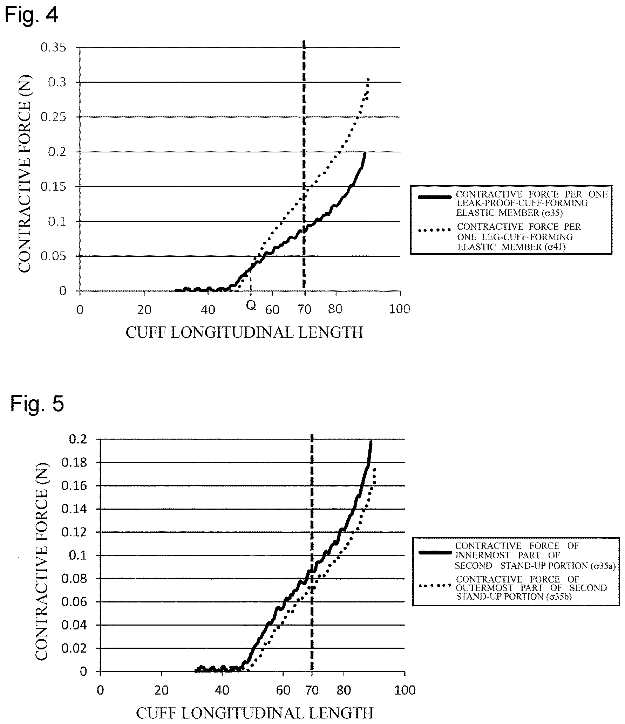

In particular, when the cuff longitudinal length is in a range of less than the particular value (i.e., when the cuff longitudinal length is relatively small), the magnitude relation "contractive force σ35 > contractive force σ41" is preferably satisfied, and when the cuff longitudinal length is in a range of more than the particular value and not more than 100 (i.e. when the cuff longitudinal length is relatively large), the magnitude relation "contractive force σ35 < contractive force σ41" is preferably satisfied. The graph shown in Fig. 4 is an example of such preferred embodiments. In the graph shown in Fig. 4, each of the contractive force per one elastic member in the second stand-up portion 35 in the leak-proof cuff 3, σ35, and the contractive force per one elastic member in the leg cuff 41, σ41, increases as the elongation increases, but their increasing rates differ from each other. Specifically, the contractive force σ35 has a small gradient in the graph and therefore a small increasing rate, and thus the contractive force σ35 does not greatly increase even when the cuff longitudinal length greatly increases. In contrast, the contractive force σ41 has a large gradient in the graph and therefore a large increasing rate, and thus the contractive force σ41 greatly increases when the cuff longitudinal length greatly increases. As a result, at a cuff longitudinal length of a particular value Q, the contractive force σ35 is equal to the contractive force σ41, and within a cuff longitudinal length range of more than the particular value Q, the magnitude relation between the contractive force σ35 and the contractive force σ41 reverses, so that the contractive force σ41 is larger than the contractive force σ35. When the contractive force σ35 and the contractive force σ41 satisfy the relation as shown in Fig. 4, the second stand-up portions 35 contract before the leg cuffs 41 contract. As a result, the leak-proof cuffs 3 (stand-up portions 32) have more favorable standing-up behavior, and the leg cuffs 41 can come into close contact with the legs of a wearer with well-fitting.

When the contractive force σ35 and the contractive force σ41 satisfy the relation as shown in Fig. 4, the particular value Q of the cuff longitudinal length at which the magnitude relation between the contractive force σ35 and the contractive force σ41 reverses (the cuff longitudinal length at which the contractive force σ35 is equal to the contractive force σ41) is preferably less than 70. When such a condition is satisfied, the advantageous effect by satisfying both the conditions (A) and (B) is further reliably exhibited. The particular value Q is preferably a numerical value of 30 or more and 69 or less and more preferably 40 or more and 60 or less.

For satisfying the relation between the contractive force σ35 and the contractive force σ41 shown in Fig. 4, the leak-proof-cuff-forming elastic member 36 may have a smaller diameter and a higher extension rate than the leg-cuff-forming elastic member 42, for example. In such a condition, the ratio of the diameter of the leak-proof-cuff-forming elastic member 36 to the diameter of the leg-cuff-forming elastic member 42 (diameter of elastic member 36/diameter of elastic member 42) is preferably 0.2 or more and more preferably 0.3 or more and is preferably 0.9 or less and more preferably 0.8 or less.

At a cuff longitudinal length of 70, the contractive force of a part corresponding to the laterally innermost member (member indicated by the sign 36a in the drawing) of a plurality of (in the present embodiment, four) leak-proof-cuff-forming elastic members 36 in the second stand-up portion 35 (hereinafter also referred to as "innermost part of the second stand-up portion"), σ₇₀35a, is preferably equal to or larger than the contractive force of a part corresponding to the laterally outermost member (member indicated by the sign 36b in the drawing) of the plurality of leak-proof-cuff-forming elastic members 36 in the second stand-up portion 35 (hereinafter also referred to as "outermost part of the second stand-up portion), σ₇₀35b. Herein, a "part corresponding to a leak-proof-cuff-forming elastic member 36a or 36b in a second stand-up portion 35" means a part overlapping with the elastic member 36a or 36b in the second stand-up portion 35 in a plan view. At a cuff longitudinal length of the second stand-up portion 35 of 70, when the contractive force of the innermost part of the second stand-up portion (part with the laterally innermost leak-proof-cuff-forming elastic member 36a), σ₇₀35a, is equal to or larger than the contractive force of the outermost part of the second stand-up portion (part with the laterally outermost leak-proof-cuff-forming elastic member 36b), σ₇₀35b as described above, the whole surface of the second stand-up portions 35 comes into contact with the skin of a wearer, to thereby further enhance well-fitting of the leak-proof cuffs 3.

Fig. 5 is an example of a graph showing the relation between the cuff longitudinal length and the contractive force (σ35a, σ35b), for the innermost part of the second stand-up portion (part overlapping with the elastic member 36a in a plan view) and the outermost part of the second stand-up portion (part overlapping with the elastic member 36b in a plan view). As described above, the magnitude relation "contractive force of innermost part of the second stand-up portion, σ35a ≥ contractive force of outermost part of the second stand-up portion, σ35b" is preferably satisfied at least at a cuff longitudinal length of 70, and the magnitude relation is more preferably satisfied constantly regardless of cuff longitudinal length as in the example shown in Fig. 5.

The ratio of the contractive force σ₇₀35a to the contractive force σ₇₀35b (σ₇₀35a/σ₇₀35b) is preferably 1 or more and more preferably 1.05 or more and is preferably 3 or less and more preferably 2 or less.

The contractive force σ₇₀35a (contractive force of the part corresponding to the laterally innermost member of the plurality of leak-proof-cuff-forming elastic members 36 in the second stand-up portion 35 at a cuff longitudinal length of 70) is preferably 0.03 N or more and more preferably 0.06 N or more and is preferably 0.15 N or less and more preferably 0.12 N or less.

The contractive force σ₇₀35b (contractive force of the part corresponding to the laterally outermost member of the plurality of leak-proof-cuff-forming elastic members 36 in the second stand-up portion 35 at a cuff longitudinal length of 70) is preferably 0.02 N or more and more preferably 0.05 N or more and is preferably 0.14 N or less and more preferably 0.11 N or less.

The present invention has been described hereinabove, but the present invention is not limited to the above embodiments and can be appropriately modified without departing from the scope of the present invention.

For example, in the diaper 1 shown in Fig. 2, the leak-proof cuff 3 is folded outward (the first stand-up portion 34 extends inward in the lateral direction Y from the base end portion 31 to the folding portion 33, and the second stand-up portion 35 extends outward in the lateral direction Y from the folding portion 33). Alternatively, a leak-proof cuff 3 may be folded inward (a first stand-up portion 34 may extend outward in the lateral direction Y from a base end portion 31 to a folding portion 33, and a second stand-up portion 35 may extend inward in the lateral direction Y from the folding portion 33). However, as described above, the leak-proof cuff 3 is preferably folded outward because effects specific to the embodiment are exhibited.

The absorbent article of the present invention widely encompasses articles used for absorption of body fluids excreted from a human body (such as urine, menstrual blood, loose feces, and sweat) and can encompass, in addition to such open type disposable diapers as in the above embodiments, pull-on disposable diapers, incontinence pads, sanitary napkins, sanitary shorts, and the like. In consideration of the embodiments of the present invention, the following aspects are further disclosed.
<1> An absorbent article having a longitudinal direction corresponding to the front-rear direction of a wearer and a lateral direction orthogonal to the longitudinal direction, the absorbent article including:
   an absorbent assembly including an absorbent member,
   a pair of leak-proof cuffs extending in the longitudinal direction and provided on a skin-facing surface of the absorbent assembly while a gap is interposed therebetween,
   side sheets extending outward in the lateral direction from side edges along the longitudinal direction of the absorbent member, and
   a pair of leg cuffs provided outside the pair of leak-proof cuffs in the lateral direction and in regions corresponding to the legs of a wearer, in which
   each of the pair of leak-proof cuffs includes the side sheet, and has, in the lateral direction, a base end portion at which the side sheet is fixed to another member and a stand-up portion that is a portion of the side sheet and stands toward a wearer from the base end portion as a starting point,
   the stand-up portion is folded along a folding portion extending in the longitudinal direction and includes a first stand-up portion extending from the base end portion to the folding portion and a second stand-up portion extending from the folding portion to a free end of the stand-up portion and to be placed closer to the skin of a wearer than the first stand-up portion; in the second stand-up portion, a plurality of leak-proof-cuff-forming elastic members are arranged in the lateral direction in a stretchable state in the longitudinal direction,
   the leg cuff includes the side sheet and a plurality of leg-cuff-forming elastic members provided on the side sheet; the plurality of leg-cuff-forming elastic members are arranged in the lateral direction in a stretchable state in the longitudinal direction and located outside the base end portion in the lateral direction,
   the contractive force per one leak-proof-cuff-forming elastic member in the second stand-up portion at a cuff longitudinal length of 70 is smaller than the contractive force per one leg-cuff-forming elastic member in the leg cuff at a cuff longitudinal length of 70, and
   the contractive force of the whole second stand-up portion at a cuff longitudinal length of 70 is larger than the contractive force of the whole leg cuff at a cuff longitudinal length of 70,
   where the cuff longitudinal length is a length of each of the leak-proof cuff and the leg cuff in the longitudinal direction, and the cuff longitudinal length of the leak-proof cuff and the cuff longitudinal length of the leg cuff in a fully stretched state are each defined as 100.
<2> The absorbent article as set forth in clause <1>, in which the ratio of the contractive force per one leak-proof-cuff-forming elastic member at a cuff longitudinal length of 70 to the contractive force per one leg-cuff-forming elastic member at a cuff longitudinal length of 70 is 0.2 or more and 0.99 or less and preferably 0.3 or more and 0.9 or less.
<3> The absorbent article as set forth in clause <1> or <2>, in which the contractive force per one leak-proof-cuff-forming elastic member at a cuff longitudinal length of 70 is 0.03 N or more and 0.15 N or less and preferably 0.06 N or more and 0.12 N or less.
<4> The absorbent article as set forth in any one of clauses <1> to <3>, in which the contractive force per one leg-cuff-forming elastic member at a cuff longitudinal length of 70 is 0.04 N or more and 0.24 N or less and preferably 0.08 N or more and 0.20 N or less.

### Industrial Applicability

The present invention provides an absorbent article that includes leak-proof cuffs having strong capability to prevent lateral leakage and leg cuffs achieving well-fitting and can effectively prevent lateral leakage.

## Claims

1. An absorbent article having a longitudinal direction (X) corresponding to a front-rear direction of a wearer and a lateral direction (Y) orthogonal to the longitudinal direction (X), the absorbent article comprising:
an absorbent assembly (2) including an absorbent member (23);
a pair of leak-proof cuffs (3) extending in the longitudinal direction (X) and provided on a skin-facing surface of the absorbent assembly (2) while a gap (39) is interposed therebetween;
side sheets (30) extending outward in the lateral direction (Y) from side edges (23S) along the longitudinal direction (X) of the absorbent member (23); and
a pair of leg cuffs (41) provided outside the pair of leak-proof cuffs (3) in the lateral direction (Y) and in regions corresponding to legs of a wearer; wherein
each of the pair of leak-proof cuffs (3) includes the side sheet (30), and has, in the lateral direction (Y), a base end portion (31) at which the side sheet (30) is fixed to another member and a stand-up portion (32) that is a portion of the side sheet (30) and stands toward a wearer from the base end portion (31) as a starting point;
the stand-up portion (32) is folded along a folding portion (33) extending in the longitudinal direction and includes a first stand-up portion (34) extending from the base end portion (31) to the folding portion and a second stand-up portion (35) extending from the folding portion (33) to a free end (32a) of the stand-up portion (32) and to be placed closer to the skin of a wearer than the first stand-up portion (34); in the second stand-up portion (35), a plurality of leak-proof-cuff-forming elastic members (36) are arranged in the lateral direction (Y) in a stretchable state in the longitudinal direction (X);
the leg cuff (41) includes the side sheet (30) and a plurality of leg-cuff-forming elastic members (36) provided on the side sheet (30); the plurality of leg-cuff-forming elastic members (36) are arranged in the lateral direction (Y) in a stretchable state in the longitudinal direction (X) and located outside the base end portion (31) in the lateral direction (Y);
a contractive force per one leak-proof-cuff-forming elastic member (36) in the second stand-up portion (35) at a cuff longitudinal length of 70 is smaller than a contractive force per one leg-cuff-forming elastic member (36) in the leg cuff at a cuff longitudinal length of 70; and
a contractive force of the whole second stand-up portion (35) at a cuff longitudinal length of 70 is larger than a contractive force of the whole leg cuff (41) at a cuff longitudinal length of 70;
where the cuff longitudinal length is a length of each of the leak-proof cuff (3) and the leg cuff (41) in the longitudinal direction (X), and the cuff longitudinal length of the leak-proof cuff (3) and the cuff longitudinal length of the leg cuff (41) in a fully stretched state are each defined as 100;
wherein the contractive force is measured as defined in the description.

2. The absorbent article according to claim 1, wherein a ratio of the contractive force per one leak-proof-cuff-forming elastic member (36) at a cuff longitudinal length of 70 to the contractive force per one leg-cuff-forming elastic member (36) at a cuff longitudinal length of 70 is 0.2 or more and 0.99 or less.

3. The absorbent article according to claim 1 or 2, wherein the contractive force per one leak-proof-cuff-forming elastic member (36) at a cuff longitudinal length of 70 is 0.03 N or more and 0.15 N or less.

4. The absorbent article according to any one of claims 1 to 3, wherein the contractive force per one leg-cuff-forming elastic member (36) at a cuff longitudinal length of 70 is 0.04 N or more and 0.24 N or less.

5. The absorbent article according to any one of claims 1 to 4, wherein the ratio of the contractive force of the whole second stand-up portion (35) at a cuff longitudinal length of 70 to the contractive force of the whole leg cuff (41) at a cuff longitudinal length of 70 is more than 1 and not more than 6.

6. The absorbent article according to any one of claims 1 to 5, wherein the contractive force of the whole second stand-up portion (35) at a cuff longitudinal length of 70 is 0.1 N or more and 0.6 N or less.

7. The absorbent article according to any one of claims 1 to 6, wherein the contractive force of the whole leg cuff (41) at a cuff longitudinal length of 70 is 0.05 N or more and 0.5 N or less.

8. The absorbent article according to any one of claims 1 to 7, wherein the number of the leak-proof-cuff-forming elastic members (36) in the second stand-up portion (35) is more than the number of the leg-cuff-forming elastic members (36).

9. The absorbent article according to any one of claims 1 to 8, wherein a magnitude relation between the contractive force per one leak-proof-cuff-forming elastic member (36) in the second stand-up portion (35) and the contractive force per one leg-cuff-forming elastic member (36) in the leg cuff (41) within a cuff longitudinal length range of less than a particular value differs from that within a cuff longitudinal length range of more than the particular value and not more than 100.

10. The absorbent article according to claim 9, wherein the particular value is less than 70.

11. The absorbent article according to any one of claims 1 to 10, wherein the ratio of a diameter of the leak-proof-cuff-forming elastic member (36) in the second stand-up portion (35) to a diameter of the leg-cuff-forming elastic member (36) is 0.2 or more and 0.9 or less.

12. The absorbent article according to any one of claims 1 to 11, wherein at a cuff longitudinal length of 70, the contractive force of a part corresponding to a laterally innermost leak-proof-cuff-forming elastic member (36) in the second stand-up portion (35) is equal to or larger than the contractive force of a part corresponding to a laterally outermost leak-proof-cuff-forming elastic member (36) in the second stand-up portion (35).

13. The absorbent article according to claim 12, wherein the contractive force of the part corresponding to the laterally outermost leak-proof-cuff-forming elastic member (36) in the second stand-up portion (35) is 0.02 N or more and 0.14 N or less.

14. The absorbent article according to any one of claims 1 to 13, wherein the first stand-up portion (33) extends inward in the lateral direction from the base end portion (31) to the folding portion (33), and the second stand-up portion (35) extends outward in the lateral direction from the folding portion (33).

15. The absorbent article according to claim 14, wherein at a cuff longitudinal length of 70, the laterally outermost leak-proof-cuff-forming elastic member (36) in the second stand-up portion (35) is located laterally inside a laterally innermost leg-cuff-forming elastic member of the plurality of leg-cuff-forming elastic members (36).

## Patentansprüche

1. Absorbierender Artikel mit einer longitudinalen Richtung (X), die einer Vorn-Hinten-Richtung eines Trägers entspricht, und einer Querrichtung (Y), die orthogonal zu der longitudinalen Richtung (X) ist, wobei der absorbierende Artikel aufweist:
eine absorbierende Anordnung (2), die ein absorbierendes Element (23) enthält;
ein Paar auslaufsichere Manschetten (3), die sich in der longitudinalen Richtung (X) erstrecken und an einer der Haut zugewandten Oberfläche der absorbierenden Anordnung (2) vorgesehen sind, während ein Spalt (39) dazwischen angeordnet ist;
Seitenlagen (30), die sich in der lateralen Richtung (Y) von Seitenrändern (23S) entlang der longitudinalen Richtung (X) des absorbierenden Elements (23) nach außen erstrecken; und
ein Paar von Beinmanschetten (41), die außerhalb des Paares der auslaufsicheren Manschetten (3) in der lateralen Richtung (Y) und in Bereichen vorgesehen sind, die Beinen eines Trägers entsprechen; wobei
jedes des Paars der auslaufsicheren Manschetten (3) die Seitenlage (30) enthält und in der lateralen Richtung (Y) einen Basisendabschnitt (31), an dem die Seitenlage (30) an einem anderen Element befestigt ist, und einen Aufstehabschnitt (32) aufweist, der ein Abschnitt der Seitenlage (30) ist und vom Basisendabschnitt (31) als Ausgangspunkt zu einem Träger steht;
der Aufstehabschnitt (32) entlang eines Faltabschnitts (33), der sich in der longitudinalen Richtung erstreckt, gefaltet ist und einen ersten Aufstehabschnitt (34), der sich vom Basisendabschnitt (31) zum Faltabschnitt erstreckt, und einen zweiten Aufstehabschnitt (35) aufweist, der sich vom Faltabschnitt (33) zu einem freien Ende (32a) des Aufstehabschnitts (32) erstreckt und näher an der Haut eines Trägers als der erste Aufstehabschnitt (34) platziert werden soll; im zweiten Stehabschnitt (35) mehrere eine auslaufsichere Manschette bildende elastische Elemente (36) in der lateralen Richtung (Y) in einem dehnbaren Zustand in der longitudinalen Richtung (X) angeordnet sind;
die Beinmanschette (41) die Seitenlage (30) und mehrere eine Beinmanschette bildende elastische Elemente (36) umfasst, die auf der Seitenlage (30) vorgesehen sind; die mehreren eine Beinmanschette bildenden elastischen Elemente (36) in der lateralen Richtung (Y) in einem dehnbaren Zustand in der longitudinalen Richtung (X) angeordnet sind und sich außerhalb des Basisendabschnitts (31) in der lateralen Richtung (Y) befinden;
eine Kontraktionskraft pro einem eine auslaufsichere Manschette bildenden elastischen Element (36) im zweiten Aufstehabschnitt (35) bei einer longitudinalen Manschettenlänge von 70 kleiner ist als eine Kontraktionskraft pro einem eine Beinmanschette bildenden elastischen Element (36) in der Beinmanschette bei einer longitudinalen Manschettenlänge von 70; und
eine Kontraktionskraft des gesamten zweiten Aufstehabschnitts (35) bei einer longitudinalen Manschettenlänge von 70 größer ist als eine Kontraktionskraft der gesamten Beinmanschette (41) bei einer longitudinalen Manschettenlänge von 70;
wobei die longitudinale Manschettenlänge eine Länge sowohl der auslaufsicheren Manschette (3) als auch der Beinmanschette (41) in der longitudinalen Richtung (X) ist und die longitudinale Manschettenlänge der auslaufsicheren Manschette (3) und die longitudinale Manschettenlänge der Beinmanschette (41) in einem vollständig gedehnten Zustand jeweils als 100 definiert sind;
wobei die Kontraktionskraft wie in der Beschreibung definiert gemessen wird.

2. Absorbierender Artikel nach Anspruch 1, wobei das Verhältnis der Kontraktionskraft pro einem eine auslaufsichere Manschette bildenden elastischen Element (36) bei einer longitudinalen Manschettenlänge von 70 zur Kontraktionskraft pro einem eine Beinmanschette bildenden elastischen Element (36) bei einer longitudinalen Manschettenlänge von 70 0,2 oder mehr und 0,99 oder weniger beträgt.

3. Absorbierender Artikel nach Anspruch 1 oder 2, wobei die Kontraktionskraft pro einem eine Beinmanschette bildenden elastischen Element (36) bei einer longitudinalen Manschettenlänge von 70 0,03 N oder mehr und 0,15 N oder weniger beträgt.

4. Absorbierender Artikel nach einem der Ansprüche 1 bis 3, wobei die Kontraktionskraft pro einem eine Beinmanschette bildenden elastischen Element (36) bei einer Manschettenlänge von 70 0,04 N oder mehr und 0,24 N oder weniger beträgt.

5. Absorbierender Artikel nach einem der Ansprüche 1 bis 4, wobei das Verhältnis der Kontraktionskraft des gesamten zweiten Aufstehabschnitts (35) bei einer longitudinalen Manschettenlänge von 70 zur Kontraktionskraft der gesamten Beinmanschette (41) bei einer longitudinalen Manschettenlänge von 70 mehr als 1 und nicht mehr als 6 beträgt.

6. Absorbierender Artikel nach einem der Ansprüche 1 bis 5, wobei die Kontraktionskraft des gesamten zweiten Aufstehabschnitts (35) bei einer longitudinalen Manschettenlänge von 70 0,1 N oder mehr und 0,6 N oder weniger beträgt.

7. Absorbierender Artikel nach einem der Ansprüche 1 bis 6, wobei die Kontraktionskraft der gesamten Beinmanschette (41) bei einer longitudinalen Manschettenlänge von 70 0,05 N oder mehr und 0,5 N oder weniger beträgt.

8. Absorbierender Artikel nach einem der Ansprüche 1 bis 7, wobei die Anzahl der eine auslaufsichere Manschette bildenden elastischen Elemente (36) im zweiten Aufstehabschnitt (35) größer ist als die Anzahl der die Beinmanschette bildenden elastischen Elemente (36).

9. Absorbierender Artikel nach einem der Ansprüche 1 bis 8, wobei ein Größenverhältnis zwischen der Kontraktionskraft pro einem eine auslaufsichere Manschette bildenden elastischen Element (36) im zweiten Aufstehabschnitt (35) und der Kontraktionskraft pro einem eine Beinmanschette bildenden elastischen Element (36) in der Beinmanschette (41) innerhalb eines longitudinalen Manschettenlängenbereichs von weniger als einem bestimmten Wert von demjenigen innerhalb eines longitudinalen Manschettenlängenbereichs von mehr als dem bestimmten Wert und nicht mehr als 100 abweicht.

10. Absorbierender Artikel nach Anspruch 9, wobei der bestimmte Wert kleiner als 70 ist.

11. Absorbierender Artikel nach einem der Ansprüche 1 bis 10, wobei das Verhältnis eines Durchmessers des eine auslaufsichere Manschette bildenden elastischen Elements (36) im zweiten Aufstehabschnitt (35) zu einem Durchmesser des die Beinmanschette bildenden elastischen Elements (36) 0,2 oder mehr und 0,9 oder weniger beträgt.

12. Absorbierender Artikel nach einem der Ansprüche 1 bis 11, wobei bei einer longitudinalen Manschettenlänge von 70 die Kontraktionskraft eines Teils, der einem lateral innersten, eine auslaufsichere Manschette bildenden elastischen Element (36) im zweiten Aufstehabschnitt (35) entspricht, gleich oder größer ist als die Kontraktionskraft eines Teils, der einem lateral äußersten, eine auslaufsichere Manschette bildenden elastischen Element (36) im zweiten Aufstehabschnitt (35) entspricht.

13. Absorbierender Artikel nach Anspruch 12, wobei die Kontraktionskraft des Teils, der dem lateral äußersten, eine auslaufsichere Manschette bildenden elastischen Element (36) im zweiten Aufstehabschnitt (35) entspricht, 0,02 N oder mehr und 0,14 N oder weniger beträgt.

14. Absorbierender Artikel nach einem der Ansprüche 1 bis 13, wobei sich der erste Aufstehabschnitt (33) in der lateralen Richtung vom Basisendabschnitt (31) zum Faltabschnitt (33) nach innen erstreckt und der zweite Aufstehabschnitt (35) sich vom Faltabschnitt (33) in der lateralen Richtung nach außen erstreckt.

15. Absorbierender Artikel nach Anspruch 14, wobei bei einer longitudinalen Manschettenlänge von 70 das lateral äußerste, eine auslaufsichere Manschette bildende elastische Element (36) im zweiten Aufstehabschnitt (35) seitlich innerhalb eines lateral innersten, eine Beinmanschette bildenden elastischen Elements der mehreren der eine Beinmanschette bildenden elastischen Elemente (36) angeordnet ist.

## Revendications

1. Article absorbant ayant une direction longitudinale (X) correspondant à une direction avant-arrière d'un porteur et une direction latérale (Y) orthogonale à la direction longitudinale (X), ledit article absorbant comprenant :
un ensemble absorbant (2) comprenant un élément absorbant (23) ;
une paire de revers étanches (3) s'étendant dans la direction longitudinale (X) et prévus sur une surface de l'ensemble absorbant (2) opposée à la peau, un espace (39) étant intercalé entre les deux ;
des feuilles latérales (30) s'étendant vers l'extérieur dans la direction latérale (Y) à partir des bords latéraux (23S) dans la direction longitudinale (X) de l'élément absorbant (23) ; et
une paire de revers de jambe (41) situés extérieurement à la paire de revers étanches (3) dans la direction latérale (Y) et dans les zones correspondant aux jambes d'un porteur ; où
chacune des paires de revers étanches (3) comprend la feuille latérale (30) et présente, dans la direction latérale (Y), une partie d'extrémité de base (31) où la feuille latérale (30) est fixée à un autre élément et une partie redressée (32) qui est une partie de la feuille latérale (30) et est dressée vers un porteur à partir de la partie d'extrémité de base (31) en tant que point initial ;
la partie redressée (32) est repliée le long d'une partie de pliage (33) s'étendant dans la direction longitudinale et comprend une première partie redressée (34) s'étendant de la partie d'extrémité de base (31) à la partie de pliage et une deuxième partie redressée (35) s'étendant de la partie de pliage (33) à une extrémité libre (32a) de la partie redressée (32) et à mettre en place plus près de la peau d'un porteur que la première partie redressée (34) ; dans la deuxième partie redressée (35), une pluralité d'éléments élastiques (36) formant le revers étanche sont disposés dans la direction latérale (Y) en état d'extensibilité dans la direction longitudinale (X) ;
le revers de jambe (41) comprend la feuille latérale (30) et une pluralité d'éléments élastiques (36) formant le revers de jambe prévus sur la feuille latérale (30) ; la pluralité d'éléments élastiques (36) formant le revers de jambe sont disposés dans la direction latérale (Y) en état d'extensibilité dans la direction longitudinale (X) et disposés extérieurement à la partie d'extrémité de base (31) dans la direction latérale (Y) ;
une force de contraction par un élément élastique (36) formant le revers étanche dans la deuxième partie redressée (35) à une longueur longitudinale de revers égale à 70 est inférieure à une force de contraction par un élément élastique (36) formant le revers de jambe dans le revers de jambe à une longueur longitudinale de revers égale à 70 ; et
la force de contraction de l'ensemble de la deuxième partie redressée (35) à une longueur longitudinale de revers égale à 70 est supérieure à la force de contraction de l'ensemble du revers de jambe (41) à une longueur longitudinale de revers égale à 70 ;
où la longueur longitudinale de revers est une longueur du revers étanche (3) et du revers de jambe (41) dans la direction longitudinale (X), et la longueur longitudinale du revers étanche (3) et la longueur longitudinale du revers de jambe (41) en état complètement étiré sont toutes deux définies comme étant égale à 100 ;
la force de contraction est mesurée comme indiqué dans la description.

2. Article absorbant selon la revendication 1, où le rapport entre la force de contraction par un élément élastique (36) formant le revers étanche à une longueur longitudinale de revers égale à 70 et la force de contraction par un élément élastique (36) formant le revers de jambe à une longueur longitudinale de revers égale à 70 est égal ou supérieur à 0,2 et inférieur ou égal à 0,99.

3. Article absorbant selon la revendication 1 ou la revendication 2, où la force de contraction par un élément élastique (36) formant le revers étanche à une longueur longitudinale de revers égale à 70 est égale ou supérieure à 0,03 N et inférieure ou égale à 0,15 N.

4. Article absorbant selon l'une des revendications 1 à 3, où la force de contraction d'un élément élastique (36) formant le revers à une longueur longitudinale de revers égale à 70 est égale ou supérieure à 0,04 N et inférieure ou égale à 0,24 N.

5. Article absorbant selon l'une des revendications 1 à 4, où le rapport entre la force de contraction de l'ensemble de la deuxième partie redressée (35) à une longueur longitudinale de revers égale à 70 et la force de contraction de l'ensemble du revers de jambe (41) à une longueur longitudinale de revers égale à 70 est supérieur à 1 et inférieur ou égal à 6.

6. Article absorbant selon l'une des revendications 1 à 5, où la force de contraction de l'ensemble de la deuxième partie redressée (35) à une longueur longitudinale de revers égale à 70 est égale ou supérieure à 0,1 N et inférieure ou égale à 0,6 N.

7. Article absorbant selon l'une des revendications 1 à 6, où la force de contraction du revers de jambe (41) dans son ensemble à une longueur longitudinale de revers égale à 70 est égale ou supérieure à 0,05 N et inférieure ou égale à 0,5 N.

8. Article absorbant selon l'une des revendications 1 à 7, où le nombre d'éléments élastiques (36) formant le revers étanche dans la deuxième partie redressée (35) est supérieur au nombre d'éléments élastiques (36) formant le revers de jambe.

9. Article absorbant selon l'une des revendications 1 à 8, où un rapport de grandeur entre la force de contraction par élément élastique (36) formant le revers étanche dans la deuxième partie redressée (35) et la force de contraction par élément élastique (36) formant le revers de jambe dans le revers de jambe (41) à l'intérieur d'une plage de longueur longitudinale de revers inférieure à une valeur particulière diffère de celui à l'intérieur d'une plage de longueur longitudinale de revers supérieure à la valeur particulière et ne dépassant pas 100.

10. Article absorbant selon la revendication 9, où la valeur particulière est inférieure à 70.

11. Article absorbant selon l'une des revendications 1 à 10, où le rapport entre le diamètre de l'élément élastique (36) formant le revers étanche dans la deuxième partie redressée (35) et le diamètre de l'élément élastique (36) formant le revers de jambe est égal ou supérieur à 0,2 et inférieur ou égal à 0,9.

12. Article absorbant selon l'une des revendications 1 à 11, où, à une longueur longitudinale de revers égale à 70, la force de contraction d'une partie correspondant à un élément élastique (36) formant le revers étanche latéralement le plus intérieur dans la deuxième partie redressée (35) est égale ou supérieure à la force de contraction d'une partie correspondant à un élément élastique (36) formant le revers étanche latéralement le plus extérieur dans la deuxième partie redressée (35).

13. Article absorbant selon la revendication 12, où la force de contraction de la partie correspondant à l'élément élastique (36) formant le revers étanche le plus à l'extérieur latéralement dans la deuxième partie redressée (35) est égale ou supérieure à 0,02 N et inférieure ou égale à 0,14 N.

14. Article absorbant selon l'une des revendications 1 à 13, où la première partie redressée (33) s'étend vers l'intérieur dans la direction latérale depuis la partie d'extrémité de la base (31) jusqu'à la partie de pliage (33), et la deuxième partie redressée (35) s'étend vers l'extérieur dans la direction latérale à partir de la partie de pliage (33).

15. Article absorbant selon la revendication 14, où, à une longueur longitudinale de revers égale à 70, l'élément élastique (36) formant le revers étanche le plus à l'extérieur latéralement dans la deuxième partie redressée (35) est situé latéralement à l'intérieur d'un élément élastique de formation de revers de jambe le plus à l'intérieur latéralement de la pluralité d'éléments élastiques (36) formant le revers de jambe.
